# EUROPEAN PATENT SPECIFICATION

(11) **EP 4 147 740 B1**
(45) Date of publication and mention of the grant of the patent: **18.03.2026**
(21) Application number: 22192370.9
(22) Date of filing: 26.08.2022
(51) Int. Cl.: A61M 25/00

(54) **ANNEALING OF DISCRETE SECTIONS OF A REINFORCEMENT LAYER TO MODULATE STIFFNESS OF A CATHETER**
GLÜHEN VON EINZELNEN ABSCHNITTEN EINER VERSTÄRKUNGSSCHICHT ZUR MODULATION DER STEIFIGKEIT EINES KATHETERS
RECUIT DE SECTIONS DISCRÈTES D'UNE COUCHE DE RENFORCEMENT POUR MODULER LA RIGIDITÉ D'UN CATHÉTER

(30) Priority: 29.08.2021 US 202117446297
(43) Date of publication of application: 15.03.2023
(73) Proprietor: DePuy Synthes Products, Inc., Raynham, MA 02767 (US)
(72) Inventor: RUIZ, Andres, Raynham, 02767 (US); POSADA, Jorge, Jr., Raynham, 02767 (US)
(74) Representative: Carpmaels & Ransford LLP

(56) References cited:
- WO-A1-2006/133959
- WO-A1-2006/135774
- US-A1- 2018 310 957
- US-B1- 7 905 877

## Description

### BACKGROUND OF THE INVENTION

### Field of the Invention

The present invention relates to a catheter used during a medical procedure. For instance, the present invention is applicable to a catheter highly trackable (i.e., the ease by which the catheter follows the guide wire) through tortuous vasculature during an intraluminal, minimally invasive, endovascular medical procedure. The present inventive catheter includes a reinforcement layer a localized or discrete section of which is annealed altering its crystalline structure to modulate (e.g., increase) the stiffness, as desired.

### Description of Related Art

Catheters are widely used in various medical treatments and procedures, for example, intracranial ischemic stroke treatment. Medical treatment procedures often require high trackability or navigation of the catheter through tortuous pathways (e.g., Ophthalmic artery, brachial artery, etc.) without damaging the tissue. It is desirable to provide a highly trackable catheter with increased stiffness without diminishing softness and flexibility of the distal end allowing navigation through tortuous vasculature to a target site in the body without damaging the tissue.

The present invention is a highly trackable catheter with modulated (e.g., increased) stiffness, as desired, along a discrete annealed section thereof while maintaining the softness and flexibility of the distal end able to navigate tortuous pathways without damaging the tissue.

EP 1551489 A1 relates to a multi-layer catheter for navigation to remote locations within the body, comprising a liner layer, a braid layer and an outer jacket.

### Summary of the Invention

The invention is defined in the independent claims. Embodiments of the invention are defined in the dependent claims.

### Brief Description of the Drawing

The foregoing and other features of the present invention will be more readily apparent from the following detailed description and drawings of illustrative of the invention wherein like reference numbers refer to similar elements throughout the several views and in which:

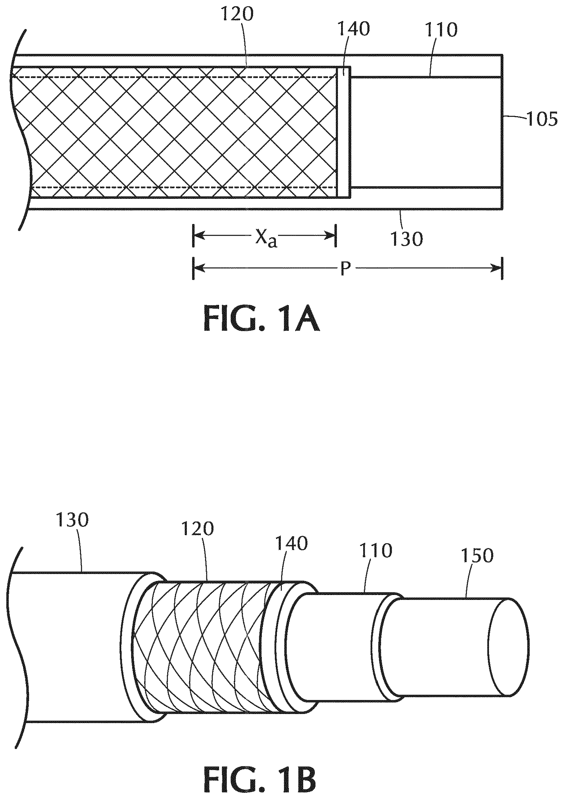

Figure 1A is a side view depicting the distal end of an exemplary catheter in accordance with the present invention including a uniform (i.e., without any transition) reinforcement layer (e.g., braided layer) a discrete section of which is annealed to modulate (e.g., increase) stiffness, as desired;
Figure 1B is a partial cut away view of distal end of the exemplary catheter of Figure 1A illustrating the three different layers (e.g., inner liner; reinforcement layer (e.g., braided layer); outer jacket) formed about a cylindrical mandrel;

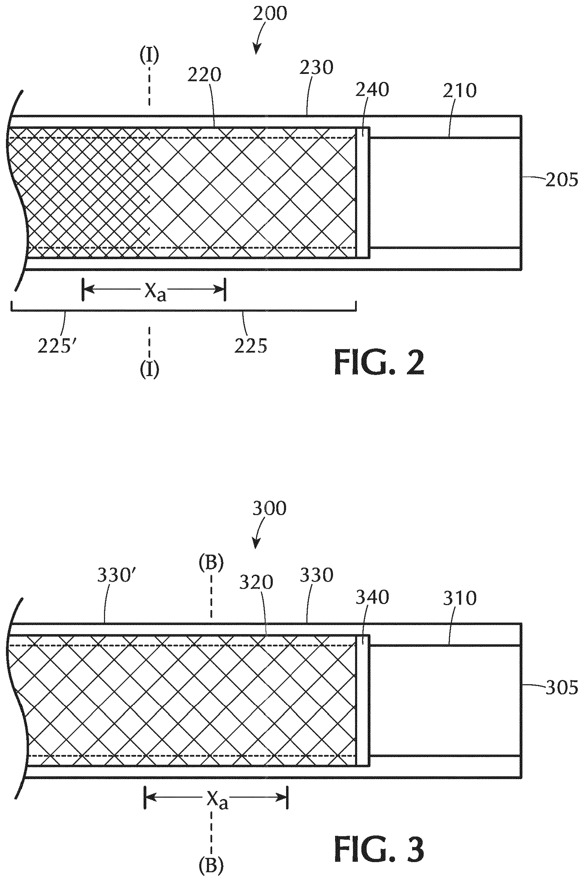

Figure 2 is a side view depicting the distal end of an exemplary catheter in accordance with the present invention including a non-uniform (i.e., having a transition) reinforcement layer (e.g., braided layer) and an outer jacket; wherein a discrete annealed section of the reinforcement layer includes the transition; and
Figure 3 is a side view depicting the distal end of an exemplary catheter in accordance with the present invention having a uniform (i.e., without or free of any transition) reinforcement layer (e.g., braided layer) a discrete section of which is annealed to modulate (e.g., increase) the stiffness, as desired, and an outer jacket comprising two different polymer layers arranged side-by-side abutting one another along an anticipated interface; wherein the annealed section of the braided layer spans the anticipated interface between the two different polymer layers comprising the outer jacket.

### Detailed Description of the Invention

The terms "distal" or "proximal" are used in the following description with respect to a position or direction relative to the treating physician or medical interventionalist. "Distal" or "distally" are a position distant from or in a direction away from the physician or interventionalist. "Proximal" or "proximally" or "proximate" are a position near or in a direction toward the physician or medical interventionist.

The present inventive intraluminal endovascular catheter comprises multiple layers including, at least, the following: an inner liner (e.g., a lubricious material such as Polytetrafluoroethylene (PTFE)); a reinforcement layer (e.g., radial crisscross or lattice braiding made of biocompatible metal wires; or a coil formed from a biocompatible metal wire); and an outer jacket (e.g., a polymer such as Polyurethane, Polyethylene, Polyether block amide (PEBA)). The distal end/tip of the inner liner (representing the distal end/tip of the catheter) extends further in a distal direction than the distal edge of the reinforcement layer (i.e., the distal edge of the reinforcement layer terminates proximally of the distal tip/end of the inner liner leaving a distal section of the inner liner exposed) providing the desirable flexibility to the catheter while navigating the tortuous vasculature (e.g., Ophthalmic artery, brachial artery, etc.) to a target site (e.g., in the brain, peripheral vasculature, etc.) without damaging the tissue. Despite these advantages, such flexibility in combination with a relatively large diameter size hampers the trackability of the catheter through tortuous vasculature to the target site when force is applied to the proximal end (pushing in a distal direction). The prevent inventive catheter is highly trackable as a result of modifying/modulating the stiffness (e.g., increasing), as desired, of one or more localized discrete sections of the metal reinforcement layer (e.g., interwoven braided or coil) via annealing without diminishing the flexibility of the distal tip/end of the catheter shaft and the benefits therefrom. Annealing process herein involves heating (e.g., using a laser) a discrete or localized section of the metal wires comprising the reinforcement layer (e.g., interwoven braid or coil) changing its crystalline structure to achieve the desired stiffness. A marker band (e.g., radiopaque marker) is placed over or immediately adjacent to the distal edge of the reinforcement layer. An outer jacket is place about those assembled layers (including the inner liner, the reinforcement layer, the marker band) forming an assembled structure the individual layers of which are reflowed together (via the application of heat) producing an integral structure composite catheter shaft.

Exemplary embodiments or configurations of the present inventive catheter in which the stiffness is modified (increased) along one or more discrete annealed sections of the reinforcement layer are described in detail below. The different embodiments discussed herein represent non-limiting variations in one or more of those basic layers (e.g., inner liner, reinforcement layer, and/or outer jacket) comprising the composite catheter shaft.

In the description certain references are made to non-SI units (inches). These are to be translated to corresponding SI units (mm) using the factor: 1 inch = approximately 25.4 mm. Figure 1A is a side view of the distal end of the present inventive catheter 100 including an inner liner (e.g., PTFE liner) 110; a metal reinforcement layer 120 (e.g., radial crisscross braiding of biocompatible metal wires (e.g., stainless steel) that is uniform (i.e., non-transitioning or unchanging) in characteristics or properties (e.g., picks per inch (PPI) of the braid); and an outer jacket 130 comprising a polymer material, as shown in Figure 1B. As previously mentioned, the distal non-reinforced portion of the catheter 100 comprising the exposed (non-reinforced) inner liner 110 extending from the distal edge/side of the reinforcement layer 120 to the distal/end tip 105 is flexible able to navigate through the tortuous vasculature to the target site without damaging the tissue. The stiffness of the metal reinforcement layer 120 along a discrete section (Xa) of the catheter is modulated or modified (e.g., increased) by annealing. Specifically, the discrete section (Xa) of the metal wire lattice comprising the reinforcement layer 120 is heated to alter its crystalline structure to achieve the desired stiffness. The number of discrete annealed section(s) (Xa) and the axial length of each section may be selected, as desired. In the case of multiple discrete annealed sections (Xa), the axial length of each section may, but need not necessarily, be the same. The annealed section (Xa) may be located with its proximal side/edge at a position (p), wherein p is in a range from 0 cm < p ≤ approximately 300 cm in an axial direction starting from the distal end/tip 105 of the catheter. In a distal direction, the distal edge of the braiding layer 120 terminates prior to the distal end/tip 105 and the annealing section (Xa) of the braided layer 120 is present only on the proximal side/face of the marker band 140 maintaining the flexibility of the remaining non-reinforced (exposed) distal section from the distal edge/side of the marker band 140 and extending in a distal direction including the distal end/tip 105 able to navigate the tortuous vasculature without damaging the tissue.

By way of example, in Figure 1A the proximal side/edge of the annealed section (Xa) may be located at an axial distance (p) of approximately 20 cm in a proximal direction from the distal end/tip 105 of the intraluminal endovascular catheter; and the longitudinal/axial length of the annealed section (Xa) spans approximately 4 cm in a distal direction (i.e., the distal side/edge of the annealed section (Xa) is located approximately 16 cm in a proximal direction from the distal end/tip 105).

Figure 1B is a cutaway view depicting the arrangement of the various layers disposed about a mandrel 150 during assembly of the catheter of Figure 1A. During manufacture the inner liner 110 (e.g., PTFE layer or tube) is positioned about the straight (uniform diameter) cylindrical mandrel 150. Next, the uniform (i.e., non-transitioning or unchanging in characteristics or properties) braided layer 120 is placed about the inner liner 110. In this regard, the mandrel 150 may be woven/braided over (e.g., wound about the outer surface of the mandrel) or the braid may be produced on a separate mandrel and the finished product then slid onto the mandrel 150. A discrete annealed section (Xa) of the distal portion of the braided layer 120 is subject to annealing (i.e., heating to change/alter/modify/modulate the crystalline structure to achieve the desired stiffness). The marker band 140 (e.g., radiopaque material) is placed over or positioned immediately adjacent to a distal edge of the reinforcement layer. Thereafter, the assembled catheter shaft (including the reinforced layer (a discrete portion of which represents the annealed section), the marker band, and the exposed distal portion of the inner liner extending to the distal end/tip) from the proximal end/tip to the opposite distal end/tip is encased in an outer jacket made of a polymer material. The multiple assembled layers are then reflowed together and the sacrificial mandrel is withdrawn (slid out) resulting in the integral composite catheter shaft with an axial/longitudinal lumen defined therethrough formed by the mandrel.

In accordance with this first inventive catheter 100 the annealed section (Xa) of the braided layer 120 may be selected, as desired, so long as the proximal edge/side of the annealed section (Xa) is at a position (p), wherein p is in a range from approximately 0 cm < p ≤ 300 cm, as measured in an axial direction starting from the distal end/tip 105 of the intraluminal endovascular catheter 100.

A second embodiment is depicted in the side view of Figure 2 which differs from that of Figure 1A in that the reinforcement layer 220 is non-uniform, i.e., there is a transition/change/variation in the reinforcement layer in one or more parameters such as pic count/pitch or shape of the wire (e.g., flat vs. round). In Figure 2 the reinforcement layer is a radial crisscross braiding of metal wires having at least one braid/pitch transition (e.g., at least one variation of the pic count or pitch (i.e., the number of strand crossovers per inch, or other lineal measure), typically represented by the number of pics per inch (PPI) of the braid). Variable flexibility over the axial length of the braid may be achieved by varying the pic count/pitch (e.g., PPI) and/or shape of the wire. Metal density/wire count changes flexibility. Specifically, the higher the density/pic count the stiffer the structure whereas the lower the density/pic count the more flexible (softer) the structure in relation thereto.

In the illustrated example, braided layer 220 comprises a single interface (I) representing a transition or variation in characteristic or properties along its axial length. For instance, a first portion 225' of the braided layer between the proximal side/edge and the interface (I) has a first greater pic count (stiffer) relative to that of a second portion 225 of the braided layer between the interface (I) and the distal edge/side having a second lesser pic count (softer). In this way, the first portion of the braid 225' having the first higher pic count is desirably stiffer relative to the second portion of the braid 225 having the second lower pic count which is softer.

Similar to that of the embodiment in Figure 1B, the various layers are disposed about a mandrel during assembly of the catheter of Figure 2. During manufacture an inner liner 210 (e.g., PTFE layer or tube) is positioned about a straight (uniform diameter) cylindrical mandrel 250. The non-uniform (having at least one transition in property or characteristic at an interface) braided layer 220 is positioned/wrapped about the inner liner 210. Specifically, the reinforcement layer may be braided/wrapped directly about the mandrel 250 or produced separately and slid into position onto the mandrel 250. In the example illustrated in Figure 2, braided layer 220 has a single transition in pic count from a first pic count region 225' to a second pic count region 225 at an interface ("I") between the two regions. By way of example, 90 ppi is provided in the second pic count region 225 (closest to the distal edge), whereas 120 ppi is set in the first pic count region 225' (closest to the proximal edge). Values for pic count of each region may be selected, as desired, depending on the desired properties or characteristics of each region of the reinforcement layer so long as the wire count/density differs between the two regions. Annealed section (Xa) of the braided layer 220 subject to annealing (to change the crystalline structure to achieve the desired stiffness) spans, extends, incorporates, includes, coincides with the transition interface (I). In the example, the annealed section (Xa) has a proximal side/face/edge that is a distance (p) approximately 20 cm from the distal end/tip 205 and the annealed section (Xa) of the braided layer 220 is approximately 4 cm in length in an axial direction. In an axial direction, a midpoint of the annealed section (Xa) of the braided layer 220 and transition interface (I) are aligned with one another so that starting from the transition interface (I) the annealed section (Xa) extends approximately 2 cm in opposing directions (proximal and distal directions). It is noted that the transition interface (I) and midpoint of the annealed section (Xa) need not be aligned with one another so that the axial length of the annealed section in opposing axial directions from the transition interface (I) differs. Marker band 250 (e.g., radiopaque marker) is placed immediately adjacent to or assembled over the distal edge/side of the braided layer 220 securing the free ends of the interwoven wires in place against the inner liner 210. Thereafter, the interim assembled catheter shaft (representing the braided layer (including the annealed section), the marker band, and the exposed (non-reinforced) distal portion of the inner liner 210 extending to the distal end/tip 205) from the proximal end to the opposite distal end is encased in an outer jacket 230 made of a polymer material (e.g., a single polymer material or different polymer materials that are co-extruded or continuously extruded). Lastly, the assembled various layers are reflowed together by applying heat to produce the integral composite catheter shaft. Thus, with this second inventive catheter 200 the annealed section (Xa) of the braided layer 220 may be selected, as desired, to meet the following conditions: (i) the proximal edge/side of the annealed section (Xa) is at a position (p), wherein 0 cm < p ≤ 300 cm, as measured in an axial direction starting from the distal end/tip 205 of the catheter 200; and (ii) the annealed section (Xa) incorporates, spans, includes, or covers the transition interface (I) of the braided layer 220.

Yet a third configuration of the present inventive catheter 300 represented in Figure 3 has a uniform reinforcement layer 320 (i.e., non-transitioning or unchanging in characteristics or properties (e.g., picks per inch (PPI) of the braid/pitch and/or shape of the wire)); and an outer jacket (330, 330') comprising two different polymer layers arranged side-by-side one after **the other** (in series) in an axial direction defining a boundary ("B") therebetween where the two layers abut (physically contact) one another without overlap. During manufacture of the catheter 300, the two different polymer materials comprising the outer jacket are applied after annealing of the discrete annealed section (Xa) of the reinforcement layer 320, hence prior to the two different outer polymer layers (330, 330') being applied, the boundary ("B") therebetween is thus referred to as "expected" or "anticipated." That discrete section (Xa) of the reinforcement layer 320 that is subject to annealing incorporates, spans, includes or coincides with the "expected" or "anticipated" boundary ("B") between the to be applied two different polymer layers (330, 330') comprising the outer jacket. In an exemplary embodiment, the different polymer layers may be the same polymer material differing in hardness. For example, the annealed section (Xa) of the braided layer 320 is approximately 4 cm in axial length while the two different polymer materials arranged axially in series includes a first outer polymer layer 330' (e.g., urethane with durometer 90A) and a second outer polymer layer 330 (e.g., urethane with durometer 70A) different from that of the first out polymer layer. Still referring to the example, the location of the "expected" or "anticipated" boundary ("B") between the to be applied two different polymer layers comprising the outer jacket is aligned with the midpoint in an axial direction of the annealed section (Xa) of the braided layer 320. Accordingly, following the example the discrete annealed section (Xa) will extend approximately 2 cm in length in opposing axial directions (proximal and distal) starting from the "expected" or "anticipated" boundary ("B") between the to be applied polymer materials (330, 330') comprising the outer jacket. It is noted that the "expected"/"anticipated" boundary ("B") and midpoint of the annealed section (Xa) need not be aligned with one another so that the length of the annealed section in opposing axial directions starting from the boundary ("B") differs. Thus, with this third inventive catheter 300 the annealed section (Xa) of the reinforcement layer 320 may be selected, as desired, to satisfy the following conditions: (i) the proximal edge/side of the annealed section (Xa) is at a position (p), wherein 0 < p ≤ 300 cm, as measured in an axial direction starting from the distal end/tip 305 of the catheter 300; and (ii) the discrete annealed section (Xa) incorporates, spans, includes, or coincides with the "anticipated"/"expected" boundary ("B") between the two different polymer layers comprising the outer jacket arranged axially in series when applied following annealing of the discrete section of the braided layer. Such polymer transition in the outer jacket allows force to be more efficiently transferred as the catheter traverses through the anatomy.

It is possible for more than one configuration to be combined together. For instance, the catheter may have a reinforcement layer with a single transition interface (as represented in Figure 2) and an outer jacket comprising two polymer layers arranged in an axial direction side-by-side one after the other, i.e., in series, abutting one another along a boundary (as shown in Figure 3). In such combination the discrete annealed section of the reinforced layer would both include the single transition interface as well as the "expected"/"anticipated" boundary between the two polymer material layers comprising the outer jacket. Transitions in the reinforcement layer and/or the outer jacket coinciding with annealed discrete sections of the reinforcement layer contribute to the trackability and durability of the catheter. Also, the illustrative examples in the drawings depict the present inventive catheter having a braided layer as the reinforcement layer but in any of the embodiments or configurations the reinforcement layer may be a coil. It is also noted that the illustrative examples shown in the figures and described in detail include only a single transition interface (I) in the braided layer and/or a single boundary (B) in outer jacket, but more than one (I) and/or (B) is contemplated. The present inventive catheter is suitable for use during an endovascular procedure but is applicable for use in other intraluminal medical procedures.

Thus, while there have been shown, described, and pointed out fundamental novel features of the invention as applied to a preferred embodiment thereof, it will be understood that various omissions, substitutions, and changes in the form and details of the systems/devices illustrated, and in their operation, may be made by those skilled in the art without departing from the scope of the invention as defined by the claims. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

## Claims

1. A catheter (200) comprising:
an inner liner (210) having a proximal end and an opposite distal end;
a reinforcement layer (220) disposed about the inner liner and having a distal edge separated a predetermined axial distance in a proximal direction from the distal end of the inner liner; the reinforcement layer having a discrete annealed section with an altered crystalline structure having modified stiffness relative to non-annealed sections of the reinforcement layer;
a marker band (240) positioned over or adjacent to the distal edge of the reinforcement layer; and
an outer jacket (230) disposed about an interim structure comprising the inner liner, the reinforcement layer, and the marker band forming an assembled structure individual components of which are reflowable together forming an integral composite catheter shaft; and
wherein the reinforcement layer is a braided layer of interwoven metal wires or a coil formed from a metal wire,, wherein the reinforcement layer has at least one transition interface in which at least one property of the reinforcement layer changes; and the discrete annealed section includes the at least one transition interface, wherein the reinforcement layer comprises a first portion (225') proximal to the at least one transition interface and a second portion (225) distal to the at least one transition interface, wherein the first portion has a greater pic count than the second portion.

2. The catheter in accordance with claim 1, wherein the at least one transition interface comprises a change in shape of the wire forming the braided layer or coil.

3. The catheter in accordance with claim 1, wherein the outer jacket comprises different material layers arranged side-by-side one after the other in series in an axial direction, wherein the different material layers abut one another along an anticipated boundary; and the discrete annealed section of the reinforcement layer coincides with the anticipated boundary.

4. The catheter in accordance with claim 3, wherein the different material layers are different polymer materials or a single polymer material having different levels of hardness.

5. A method for producing a catheter (200), the method comprising the steps of:
placing an inner liner (210) over a mandrel, the inner liner having a proximal end and an opposite distal end;
placing a reinforcement layer (220) about the inner liner so that a distal edge of the reinforcement layer is separated a predetermined distance in a proximal direction from the distal end of the inner liner leaving a distal section of the inner liner exposed;
annealing a discrete section of the reinforcement layer altering its crystalline structure to modify stiffness relative to non-annealed sections of the reinforcement layer;
placing a marker band (240) over or immediately adjacent the distal edge of the reinforcement layer; and
placing an outer jacket (230) about an interim structure including the inner liner, the reinforcement layer, and the marker band to form an assembled structure; and
applying heat to reflow together individual components of the assembled structure producing an integral composite catheter shaft; and
wherein the reinforcement layer is a braided layer of interwoven metal wires or a coil formed from a metal wire, and wherein the reinforcement layer has at least one transition interface in which at least one property of the reinforcement layer changes; and the discrete annealed section includes the at least one transition interface, wherein the reinforcement layer comprises a first portion (225') proximal to the at least one transition interface and a second portion (225) distal to the at least one transition interface, wherein the first portion has a greater pic count than the second portion.

6. The catheter in accordance with claim 1, wherein a proximal face of the discrete annealed section is located at an axial position **(p),** wherein 0 cm < p ≤ approximately 300 cm in a proximal direction starting from the distal end of the inner liner.

7. The method in accordance with claim 5, wherein a proximal face of the discrete annealed section is located at an axial position (p), wherein 0 cm < p ≤ approximately 300 cm in a proximal direction starting from the distal end of the inner liner.

8. The method in accordance with claim 5, wherein the at least one transition interface comprises a change in shape of the wire forming the braided layer or coil.

9. The method in accordance with claim 5, wherein the outer jacket comprises different material layers arranged side-by-side one after the other in series in an axial direction, wherein the different material layers abut one another along an anticipated boundary; and the discrete annealed section of the reinforcement layer coincides with the anticipated boundary.

10. The method in accordance with claim 9, wherein the different material layers are different polymer materials or a single polymer material differing in levels of hardness.

11. The catheter in accordance with claim 1, wherein the reinforcement layer has more than one discrete annealed section.

12. The method in accordance with claim 5, wherein the reinforcement layer has more than one discrete annealed section.

13. The catheter in accordance with claim 1, wherein the modified stiffness of the annealed discrete section has increased stiffness relative to the non-annealed sections of the reinforcement layer.

14. The method in accordance with claim 5, wherein the modified stiffness of the annealed discrete section has increased stiffness relative to the non-annealed sections of the reinforcement layer.

## Patentansprüche

1. Katheter (200) umfassend:
eine innere Auskleidung (210), die ein proximales und ein gegenüberliegendes distales Ende aufweist;
eine Verstärkungsschicht (220), die um die innere Auskleidung angeordnet ist und einen distalen Rand aufweist, der um einen vorgegebenen axialen Abstand in proximaler Richtung von dem distalen Ende der inneren Auskleidung getrennt ist; wobei die Verstärkungsschicht einen diskreten getemperten Abschnitt mit einer veränderten kristallinen Struktur mit modifizierter Steifigkeit relativ zu nicht-getemperten Abschnitten der Verstärkungsschicht aufweist;
ein Markierungsband (240), das über oder angrenzend an den distalen Rand der Verstärkungsschicht positioniert ist; und
eine äußere Umhüllung (230), die um eine Zwischenstruktur angeordnet ist, welche die innere Auskleidung, die Verstärkungsschicht und das Markierungsband umfasst und eine zusammengesetzte Struktur bildet, deren einzelne Komponenten zusammen aufschmelzbar sind und einen integralen Verbundkatheterschaft bilden; und
wobei die Verstärkungsschicht eine geflochtene Schicht aus miteinander verwobenen Metalldrähten oder eine aus einem Metalldraht gebildete Spule ist, wobei die Verstärkungsschicht mindestens eine Übergangsgrenzfläche aufweist, in der sich mindestens eine Eigenschaft der Verstärkungsschicht ändert; und der diskrete getemperte Abschnitt die mindestens eine Übergangsgrenzfläche einschließt, wobei die Verstärkungsschicht einen ersten Abschnitt (225') proximal zu der mindestens einen Übergangsgrenzfläche und einen zweiten Abschnitt (225) distal zu der mindestens einen Übergangsgrenzfläche umfasst, wobei der erste Abschnitt eine größere Pic-Zahl als der zweite Abschnitt aufweist.

2. Katheter gemäß Anspruch 1, wobei die mindestens eine Übergangsgrenzfläche eine Formänderung des Drahts umfasst, der die geflochtene Schicht oder Spule bildet.

3. Katheter gemäß Anspruch 1, wobei die äußere Umhüllung verschiedene Materialschichten umfasst, die nebeneinander nacheinander in Reihe in einer axialen Richtung angeordnet sind, wobei die verschiedenen Materialschichten entlang einer vorgesehenen Grenze aneinander anliegen; und der diskrete getemperte Abschnitt der Verstärkungsschicht mit der vorgesehenen Grenze übereinstimmt.

4. Katheter gemäß Anspruch 3, wobei die verschiedenen Materialschichten verschiedene Polymermaterialien oder ein einzelnes Polymermaterial mit verschiedenen Härtegraden sind.

5. Verfahren zum Herstellen eines Katheters (200), das Verfahren umfassend die Schritte:
Platzieren einer inneren Auskleidung (210) über einem Dorn, wobei die innere Auskleidung ein proximales Ende und ein gegenüberliegendes distales Ende aufweist;
Platzieren einer Verstärkungsschicht (220) um die innere Auskleidung, so dass ein distaler Rand der Verstärkungsschicht um eine vorgegebene Entfernung in einer proximalen Richtung von dem distalen Ende der inneren Auskleidung getrennt ist, wodurch ein distaler Abschnitt der inneren Auskleidung freigelegt bleibt;
Tempern eines diskreten Abschnitts der Verstärkungsschicht, wodurch deren kristalline Struktur verändert wird, um die Steifigkeit relativ zu nicht-getemperten Abschnitten der Verstärkungsschicht zu modifizieren;
Platzieren eines Markierungsbands (240) über oder unmittelbar angrenzend an den distalen Rand der Verstärkungsschicht; und
Platzieren einer äußeren Umhüllung (230) um eine Zwischenstruktur, die die innere Auskleidung, die Verstärkungsschicht und das Markierungsband einschließt, zur Bildung einer zusammengesetzten Struktur; und
Anwenden von Wärme, um einzelne Komponenten der zusammengebauten Struktur zusammen aufzuschmelzen, wodurch ein integraler Verbundkatheterschaft erzeugt wird; und
wobei die Verstärkungsschicht eine geflochtene Schicht aus miteinander verwobenen Metalldrähten oder eine aus einem Metalldraht gebildete Spule ist, und wobei die Verstärkungsschicht mindestens eine Übergangsgrenzfläche aufweist, in der sich mindestens eine Eigenschaft der Verstärkungsschicht ändert; und der diskrete getemperte Abschnitt die mindestens eine Übergangsgrenzfläche einschließt, wobei die Verstärkungsschicht einen ersten Abschnitt (225') proximal zu der mindestens einen Übergangsgrenzfläche und einen zweiten Abschnitt (225) distal zu der mindestens einen Übergangsgrenzfläche umfasst, wobei der erste Abschnitt eine größere Pic-Zahl als der zweite Abschnitt aufweist.

6. Katheter gemäß Anspruch 1, wobei eine proximale Fläche des diskreten getemperten Abschnitts an einer axialen Position (p) angeordnet ist, wobei 0 cm < p ≤ etwa 300 cm in einer proximalen Richtung beginnend vom distalen Ende der inneren Auskleidung.

7. Verfahren gemäß Anspruch 5, wobei eine proximale Fläche des diskreten getemperten Abschnitts an einer axialen Position (p) angeordnet ist, wobei 0 cm < p ≤ etwa 300 cm in einer proximalen Richtung beginnend vom distalen Ende der inneren Auskleidung.

8. Verfahren gemäß Anspruch 5, wobei die mindestens eine Übergangsgrenzfläche eine Formänderung des Drahts umfasst, der die geflochtene Schicht oder Spule bildet.

9. Verfahren gemäß Anspruch 5, wobei die äußere Umhüllung verschiedene Materialschichten umfasst, die nebeneinander nacheinander in Reihe in einer axialen Richtung angeordnet sind, wobei die verschiedenen Materialschichten entlang einer vorgesehenen Grenze aneinander anliegen; und der diskrete getemperte Abschnitt der Verstärkungsschicht mit der vorgesehenen Grenze übereinstimmt.

10. Verfahren gemäß Anspruch 9, wobei die verschiedenen Materialschichten verschiedene Polymermaterialien oder ein einzelnes Polymermaterial sind, das sich in den Härtegraden unterscheidet.

11. Katheter gemäß Anspruch 1, wobei die Verstärkungsschicht mehr als einen diskreten getemperten Abschnitt aufweist.

12. Verfahren gemäß Anspruch 5, wobei die Verstärkungsschicht mehr als einen diskreten getemperten Abschnitt aufweist.

13. Katheter gemäß Anspruch 1, wobei die modifizierte Steifigkeit des getemperten diskreten Abschnitts eine erhöhte Steifigkeit relativ zu den nicht-getemperten Abschnitten der Verstärkungsschicht aufweist.

14. Verfahren gemäß Anspruch 5, wobei die modifizierte Steifigkeit des getemperten diskreten Abschnitts eine erhöhte Steifigkeit relativ zu den nicht-getemperten Abschnitten der Verstärkungsschicht aufweist.

## Revendications

1. Cathéter (200) comprenant :
un revêtement interne (210) ayant une extrémité proximale et une extrémité distale opposée ;
une couche de renfort (220) disposée autour du revêtement interne et ayant un bord distal séparé à une distance axiale prédéterminée dans une direction proximale, de l'extrémité distale du revêtement interne ; la couche de renfort ayant une section recuite distincte avec une structure cristalline altérée ayant une rigidité modifiée par rapport à des sections non recuites de la couche de renfort ;
une bande de marquage (240) positionnée par-dessus le, ou adjacente au, bord distal de la couche de renfort ; et
une enveloppe externe (230) disposée autour d'une structure intermédiaire comprenant le revêtement interne, la couche de renfort et la bande de marquage formant une structure assemblée dont les composants individuels peuvent être refondus ensemble en formant une tige de cathéter composite d'un seul tenant ; et
dans lequel la couche de renfort est une couche tressée de fils métalliques entrelacés ou une bobine formée à partir d'un fil métallique, dans lequel la couche de renfort a au moins une interface de transition dans laquelle au moins une propriété de la couche de renfort change ; et la section recuite distincte comporte l'au moins une interface de transition, dans lequel la couche de renfort comprend une première partie (225') proximale par rapport à l'au moins une interface de transition et une seconde partie (225) distale par rapport à l'interface de transition, dans lequel la première partie a un plus grand nombre de croisements de fils que la seconde partie.

2. Cathéter selon la revendication 1, dans lequel l'au moins une interface de transition comprend un changement de forme du fil formant la couche tressée ou bobine.

3. Cathéter selon la revendication 1, dans lequel l'enveloppe externe comprend différentes couches de matériau agencées côte à côte les unes après les autres en série dans une direction axiale, dans lequel les différentes couches de matériau viennent en butée les unes contre les autres le long d'une limite prévue ; et la section recuite distincte de la couche de renfort coïncide avec la limite prévue.

4. Cathéter selon la revendication 3, dans lequel les différentes couches de matériau sont des matériaux polymères différents ou un unique matériau polymère ayant différents niveaux de dureté.

5. Procédé permettant de produire un cathéter (200), le procédé comprenant les étapes consistant à :
placer un revêtement interne (210) par-dessus un mandrin, le revêtement interne ayant une extrémité proximale et une extrémité distale opposée ;
placer une couche de renfort (220) autour du revêtement interne de sorte qu'un bord distal de la couche de renfort est séparé à une distance prédéterminée dans une direction proximale, de l'extrémité distale du revêtement interne en laissant une section distale du revêtement interne exposée ;
recuire une section distincte de la couche de renfort en altérant sa structure cristalline pour modifier la rigidité par rapport à des sections non recuites de la couche de renfort ;
placer une bande de marquage (240) par-dessus le, ou immédiatement adjacente au, bord de la couche de renfort ; et
placer une enveloppe externe (230) autour d'une structure intermédiaire comportant le revêtement interne, la couche de renfort et la bande de marquage pour former une structure assemblée ; et
appliquer de la chaleur pour refondre conjointement les composants individuels de la structure assemblée en produisant une tige de cathéter composite d'un seul tenant ; et
dans lequel la couche de renfort est une couche tressée de fils métalliques entrelacés ou une bobine formée à partir d'un fil métallique, et dans lequel la couche de renfort a au moins une interface de transition dans laquelle au moins une propriété de la couche de renfort change ; et la section recuite distincte comporte l'au moins une interface de transition, dans lequel la couche de renfort comprend une première partie (225') proximale par rapport à l'au moins une interface de transition et une seconde partie (225) distale par rapport à l'interface de transition, dans lequel la première partie a un plus grand nombre de croisements de fils que la seconde partie.

6. Cathéter selon la revendication 1, dans lequel une face proximale de la section recuite distincte est localisée au niveau d'une position axiale (p), dans lequel 0 cm < p ≤ approximativement 300 cm dans une direction proximale en partant de l'extrémité distale du revêtement interne.

7. Procédé selon la revendication 5, dans lequel une face proximale de la section recuite distincte est localisée au niveau d'une position axiale (p), dans lequel 0 cm < p ≤ approximativement 300 cm dans une direction proximale en partant de l'extrémité distale du revêtement interne.

8. Procédé selon la revendication 5, dans lequel l'au moins une interface de transition comprend un changement de forme du fil formant la couche tressée ou la bobine.

9. Procédé selon la revendication 5, dans lequel l'enveloppe externe comprend différentes couches de matériau agencées côte à côte les unes après les autres en série dans une direction axiale, dans lequel les différentes couches de matériau viennent en butée les unes contre les autres le long d'une limite prévue ; et la section recuite distincte de la couche de renfort coïncide avec la limite prévue.

10. Procédé selon la revendication 9, dans lequel les différentes couches de matériau sont des matériaux polymères différents ou un unique matériau polymère dont les niveaux de dureté diffèrent.

11. Cathéter selon la revendication 1, dans lequel la couche de renfort a plus d'une section recuite distincte.

12. Procédé selon la revendication 5, dans lequel la couche de renfort a plus d'une section recuite distincte.

13. Cathéter selon la revendication 1, dans lequel la rigidité modifiée de la section distincte recuite a une rigidité augmentée par rapport aux sections non recuites de la couche de renfort.

14. Procédé selon la revendication 5, dans lequel la rigidité modifiée de la section distincte recuite a une rigidité augmentée par rapport aux sections non recuites de la couche de renfort.
